# EUROPÄISCHE PATENTSCHRIFT

(11) **EP 2 004 261 B1**
(45) Veröffentlichungstag und Bekanntmachung des Hinweises auf die Patenterteilung: **25.05.2016**
(21) Anmeldenummer: 07720076.4
(22) Anmeldetag: 13.04.2007
(51) Int. Cl.: A61M 15/00

(54) **Medikamenten-Ausgabevorrichtung**
Medicament delivery device
Dispositif de distribution de médicaments

(30) Priorität: 13.04.2006 EP 06405162
(43) Veröffentlichungstag der Anmeldung: 24.12.2008
(73) Patentinhaber: BOEHRINGER INGELHEIM INTERNATIONAL GMBH, 55216 Ingelheim am Rhein (DE)
(72) Erfinder: WACHTEL, Herbert, 55218 Ingelheim am Rhein (DE); GESER, Johannes, 55218 Ingelheim am Rhein (DE); METZGER, Burkhard, P., 55218 Ingelheim am Rhein (DE); SPALLEK, Michael, 55218 Ingelheim am Rhein (DE); KRUEGER, Michael, 55218 Ingelheim am Rhein (DE); KUNZE, Hubert, 44227 Dortmund (DE); MOSER, Achim, 09130 Chemnitz (DE); MOCK, Elmar, CH-2013 Colombier (CH); LANCI, Antonino, CH-3006 Bern (CH); KLOPFENSTEIN, André, CH-2520 La Neuveville (CH)
(74) Vertreter: Frei Patent Attorneys
(86) Internationale Anmeldenummer: PCT/CH2007/000179
(87) Internationale Veröffentlichungsnummer: WO 2007/118341

(56) Entgegenhaltungen:
- WO-A-2004/091703
- DE-A1- 19 500 764
- US-B1- 6 237 590
- US-B1- 6 679 254

## Beschreibung

Die Erfindung liegt auf dem Gebiet von Medikamenten-Ausgabevorrichtungen mit einem Mehrfachmagazin, insbesondere von Inhalationsgeräten für pulverförmige Medikamente und betrifft eine Medikamenten-Ausgabevorrichtung gemäss dem Oberbegriff des unabhängigen Patentanspruchs.

Aus dem US Patent Nr. 4,627,432 ist ein Inhalationsgerät bekannt, in welchem eine Blisterpackung mit kreisförmig angeordneten Blistern durch eine Rotation weiter transportiert wird. Dabei werden die in der Blisterpackung eingebrachten Blister nacheinander in eine Inhalationsposition gebracht. Die einzelnen Blister werden zum Öffnen über einen Hebel angestochen. Dies macht die Vorrichtung relativ gross, da ein gewisser Hub für das Anstechen notwendig ist. Soll ein Öffnungsmechanismus bei bzw. in einem Mundstück angeordnet werden, wie beispielsweise im Dokument US 5,590,645 beschrieben, so ist für das Weitertransportieren und Öffnen eine relativ hohe Kraft nötig. Diese Kraft wird vorzugsweise auf einen längeren Weg verteilt, um die Bedienung des Gerätes zu erleichtern. Einen weiteren Weg bzw. eine grössere auszuführende Bewegung auf eine seitliche Bewegung eines Mundstücks zu übertragen, hätte jedoch den Nachteil, dass die einzelnen Blister in der Blisterpackung entsprechend weit voneinander entfernt sein müssten. Dies ist in Bezug auf Multidose-Inhalatoren mit einer möglichst hohen Einzeldosenzahl nachteilig und dem Bedürfnis für kleine handliche Geräte abträglich.

In mehreren Dokumenten, beispielsweise in US 6,237,590, in US 6,679,254 oder auch in DE 195 00 764 werden Medikamenten-Ausgabevorrichtungen offenbart, welche eine möglichst hohe Anzahl von Medikamentenkammern auf einem scheibenförmigen Medikamentenmagazin beschreiben. Dabei sind die Medikamentenkammern in zwei-drei konzentrischen Kreisen angeordnet, wobei sämtliche Kammern eines ersten Kreises abgefahren werden und anschliessend die Kammern des nächsten, in der Regel weiter innen liegenden, Kreises abgefahren werden. Gegebenenfalls, wie bspw. in DE 195 00 764 kann auch ein Kreis übersprungen werden. Nachteilig in diesen Vorrichtungen ist einerseits, dass eine Vorschub- bzw. Öffnungseinrichtung, die eine radiale und eine zentrale Bewegung erlauben muss, entsprechend aufwändig gestaltet sein muss. Andererseits können die einzelnen Kammern auf den konzentrischen Kreisen nur so nahe nebeneinander angeordnet sein, wie es die Öffnungseinrichtung zulässt. Eine Vorschubvorrichtung mit konstantem Vorschub und gleichzeitig hoher Kammerdichte ist mit diesen Vorrichtungen nicht möglich.

Aufgabe der Erfindung ist es, eine Medikamenten-Ausgabevorrichtung zu schaffen, mit welcher eine genügend grosse Bewegung zum Positionieren und Öffnen einer Medikamentenkammer zur Verfügung gestellt wird, bei gleichzeitig dichter Packung von Medikamentenkammern in einem Medikamentenmagazin, welche insbesondere auch für Medikamentenmagazine mit ringförmig angeordneten Medikamentenkammern geeignet ist.

Die Aufgabe wird durch die Medikamenten-Ausgabevorrichung gelöst, wie sie in den Ansprüchen definiert ist.

Die Medikamenten-Ausgabevorrichtung, vorzugsweise ein Mehrdosispulverinhalator, zur Ausgabe von Medikamenten-Einzeldosen, weist eine Vielzahl von Medikamentenkammern in einem Medikamentenmagazin auf, wobei das Magazin bzw. die darin eingebrachten Kammern in der Form einer Endlosschlaufe vorliegen. Das Medikamentenmagazin und ein Mundstück, durch welches ein Medikament von einem Patienten eingenommen, beispielsweise inhaliert werden kann, sind relativ zueinander verschiebbar, so dass sämtliche Medikamentenkammern und das Mundstück nacheinander miteinander in Übereinstimmung bringbar sind. Dabei bilden die Medikamentenkammern der Endlosschleife jeweils Gruppen, derart, dass das Mundstück verschiedene Gruppen durch einen im wesentlichen vollständigen Umgang des Medikamentenmagazins, d.h. nach einem im wesentlichen vollständigen Umgang der Schlaufe erreicht.

Ein in wesentlichen vollständiger Umgang bedeutet, dass ein Mundstück, welches sich zur Entnahme in einer anfänglichen Medikamentenkammerposition x befindet, sich nach einem im wesentlichen vollständigen Umgang vorzugsweise in einer Medikamentenkammerposition x + 1 oder x - 1 befindet. Da ein Mundstück und ein Medikamentenmagazin oder ein Gehäuse relativ zueinander verschiebbar angeordnet sind, kann sich der Umgang auf das Mundstück um das Magazin oder das Gehäuse oder auch auf eine fast vollständige Umdrehung um 360° bzw. um wenig mehr als 360° eines Medikamentenmagazins in Bezug auf ein Mundstück oder das Gehäuse beziehen.

Die erfindungsgemässe Medikamenten-Ausgabevorrichtung erlaubt nun, dass zwei aufeinanderfolgende Entnahmepositionen nicht gleich zwei nebeneinander angeordneten Kammerpositionen entsprechen. Zwischen zwei aufeinanderfolgenden Entnahmepositionen können mehrere Medikamentenkammern, typischerweise eine, zwei oder drei, angeordnet sein.

Ein zum Öffnen einer Medikamentenkammer benötigter Vorschub kann nun im wesentlichen so lang sein, wie erforderlich, ohne dass ein dazwischenliegender Bereich für den Gebrauch als Speicherplatz für Medikamentenkammern entfällt.

Dies erlaubt eine sehr hohe Medikamentenkammerdichte und damit die Schaffung eines Inhalators, welcher auch ohne Wechsel eines Medikamentenmagazins, Medikamenten-Einzeldosen für mehrere Wochen oder Monate, z. B. 30-60 Einzeldosen, beinhaltet. Dies ist bei gleichbleibender, gegebenenfalls auch geringerer Inhalatorgrösse möglich. Das Überspringen einzelner Medikamentenkammern bei einem bestimmten Umgang stellt den erforderlichen Weg zur Verfügung, der beispielsweise für einen Hub für einen Öffnungsmechanismus für eine Medikamentenkammer, z.B. mittels Stechen, erforderlich ist. Es ist aber auch möglich, die teilweise hohe Kraft, die zum Transport eines Magazins und zum Öffnen einer Kammer notwendig ist, in einen längeren Weg umzusetzen, ohne dabei Raum für Medikamentenkammern zu verlieren. Dies ist insbesondere von Vorteil bei scheibenförmigen Inhalatoren, in welchen ein Medikamentenmagazin als Ringscheibe vorliegt und die einzelnen Medikamentenkammern kreisförmig in diesem Magazin angeordnet sind. Soll nun die Transport- und Öffnungsbewegung in eine Bewegung beispielsweise eines Mundstücks entlang einem Aussenumfang der Ringscheibe integriert sein, so ist ein langer Vorschub des Mundstücks und gleichzeitig eine Anordnung von Medikamentenkammern möglich, welche dichter beieinander liegt,als eine entsprechende Vorschubdistanz.

Auch sind die Medikamenten-kammern äquidistant im Medikamentenmagazin angeordnet und ein Vorschub weist jeweils dieselbe Länge auf.

Unterschiedliche Öffnungsmechanismen, wie beispielsweise Anstechen, Peelen oder Schaben, können damit direkt in die Bewegung eines Mundstücks integriert bzw. mit der Bewegung des Mundstücks kombiniert werden.

In einer bevorzugten Ausführungsform sind Mundstück und Medikamentenmagazin nur in einer Richtung relativ zueinander verschiebbar.

Ein für die Medikamenten-Ausgabevorrichtung geeignetes Medikamentenmagazin bzw. die darin eingebrachten Medikamentenkammern liegen in einer Endlosschlaufe vor. Dies können im wesentlichen jegliche Arten von zyklischen Anordnungen von Medikamentenkammern sein. Typische Beispiele sind Ringscheiben mit kreisförmig eingebrachten Medikamentenmagazinen oder auch endlos Blisterbänder, beispielsweise in runder oder ovaler Anordnung.

Die Medikamenten-Ausgabevorrichtung weist vorzugsweise eine Anzeige zum Anzeigen in welcher Medikamentenkammer- oder Entnahmeposition sich das Mundstück befindet auf. Es ist auch möglich in einer solchen Anzeige eine Angabe über eine Gruppe von Medikamentenkammern zu machen. Dies ist insbesondere bei Vorrichtungen bzw. Medikamentenmagazinen von Vorteil, welche beispielsweise Gruppen von Medikamentenkammern aufweisen, in welchen eine sich ändernde Dosierung oder Zusammensetzung eines Medikaments befindet.

Die Anzahl der Medikamentenkammern oder Medikamenteneinzeldosen für die Vorrichtung liegt vorzugsweise in einem Bereich von 1 bis 100 oder bis 200 Einzeldosen, bevorzugt in einem Bereich von 1-60, beispielsweise zwischen 7-180 oder 14-150, z. B. 30-120, 45-100, 30, 90, 60, 120. Für Inhalationsgeräte liegt eine Maximalanzahl von Einzeldosen aus Handlichkeits- und Therapie-Gründen vorzugsweise bei 60.

Als pharmazeutisch wirksame Substanzen, Substanzformulierungen oder Substanzmischungen werden alle inhalierbaren Verbindungen eingesetzt, wie z.B. auch inhalierbare Makromoleküle, wie in EP 1 003 478 offenbart. Vorzugsweise werden Substanzen, Substanzformulierungen oder Substanzmischungen zur Behandlung von Atemwegserkrankungen eingesetzt, die im inhalativen Bereich Verwendung finden.

Die unten genannten Verbindungen können allein oder in Kombination zur Anwendung in der erfindungsgemäßen Vorrichtung gelangen. In den unten genannten Verbindungen ist **W** einen pharmakologisch, aktiver Wirkstoff und (beispielsweise) ausgewählt aus der Gruppe bestehend aus Betamimetika, Anticholinergika, Corticosteroiden, PDE4-Inhibitoren, LTD4-Antagonisten, EGFR-Hemmem, Dopamin-Agonisten, H1-Antihistaminika, PAF-Antagonisten und PI3-Kinase Inhibitoren. Weiterhin können zwei- oder dreifach Kombinationen von **W** kombiniert werden und zur Anwendung in der erfindungsgemäßen Vorrichtung gelangen. Beispielhaft genannte Kombinationen von **W** wären:
- **W** stellt ein Betamimetika dar, kombiniert mit einem Anticholinergika, Corticosteroide, PDE4-Inhibitore, EGFR-Hemmern oder LTD4-Antagonisten,
- **W** stellt ein Anticholinergika dar, kombiniert mit einem Betamimetika, Corticosteroiden, PDE4-Inhibitoren, EGFR-Hemmern oder LTD4-Antagonisten,
- **W** stellt ein Corticosteroiden dar, kombiniert mit einem PDE4-Inhibitoren, EGFR-Hemmern oder LTD4-Antagonisten
- **W** stellt ein PDE4-Inhibitoren dar, kombiniert mit einem EGFR-Hemmern oder LTD4-Antagonisten
- **W** stellt ein EGFR-Hemmern dar, kombiniert mit einem LTD4-Antagonisten.

Als Betamimetika gelangen hierbei vorzugsweise Verbindungen zur Anwendung, die ausgewählt sind aus der Gruppe bestehend aus Albuterol, Arformoterol, Bambuterol, Bitolterol, Broxaterol, Carbuterol, Clenbuterol, Fenoterol, Formoterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmefamol, Salmeterol, Soterenol, Sulphonterol, Terbutaline, Tiaramide, Tolubuterol, Zinterol, CHF-1035, HOKU-81, KUL-1248 und
- 3-(4-{6-[2-Hydroxy-2-(4-hydroxy-3-hydroxymethyl-phenyl)-ethylamino]-hexyloxy}-butyl)-benzyl-sulfonamid
- 5-[2-(5,6-Diethyl-indan-2-ylamino)-1-hydroxy-ethyl]-8-hydroxy-1Hquinolin-2-on
- 4-Hydroxy-7-[2-{[2-{[3-(2-phenylethoxy)propyl]sulphonyl}ethyl]-amino}ethyl]-2(3H)-benzothiazolon
- 1-(2-Fluor-4-hydroxyphenyl)-2-[4-(1-benzimidazolyl)-2-methyl-2-butylamino]ethanol
- 1-[3-(4-Methoxybenzyl-amino)-4-hydroxyphenyl]-2-[4-(1-benzimidazolyl)-2-methyl-2-butylamino]ethanol
- 1-[2H-5-hydroxy-3-oxo-4H-1,4-benzoxazin-8-yl]-2-[3-(4-N,N-dimethylaminophenyl)-2-methyl-2-propylamino]ethanol
- 1-[2H-5-hydroxy-3-oxo-4H-1,4-benzoxazin-8-yl]-2-[3-(4-methoxyphenyl)-2-methyl-2-propylamino] ethanol
- 1-[2H-5-hydroxy-3-oxo-4H-1,4-benzoxazin-8-yl]-2-[3-(4-n-butyloxyphenyl)-2-methyl-2-propylamino]ethanol
- 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol
- 5-Hydroxy-8-(1-hydroxy-2-isopropylaminobutyl)-2H-1,4-benzoxazin-3-(4H)-on
- 1-(4-Amino-3-chlor-5-trifluormethylphenyl)-2-tert.-butylamino)ethanol
- 6-Hydroxy-8-{1-hydroxy-2-[2-(4-methoxy-phenyl)-1,1-dimethyl-ethylamino]-ethyl}-4H-benzo[1,4]oxazin-3-on
- 6-Hydroxy-8-{1-hydroxy-2-[2-(4-phenoxy-essigsäureethylester)-1,1-dimethyl-ethylamino]-ethyl}-4H-benzo[1,4]oxazin-3-on
- 6-Hydroxy-8-{1-hydroxy-2-[2-(4-phenoxy-essigsäure)-1,1-dimethyl-ethylamino]-ethyl}-4H-benzo[1,4]oxazin-3-on
- 8-{2-[1,1-Dimethyl-2-(2,4,6-trimethylphenyl)-ethylamino]-1-hydroxyethyl}-6-hydroxy-4H-benzo[1,4]oxazin-3-on
- 6-Hydroxy-8-{1-hydroxy-2-[2-(4-hydroxy-phenyl)-1,1-dimethylethyl amino]-ethyl}-4H-benzo[1,4]oxazin-3-on
- 6-Hydroxy-8-{1-hydroxy-2-[2-(4-isopropyl-phenyl)-1,1dimethyl-ethylamino]-ethyl}-4H-benzo[1,4]oxazin-3-on
- 8-{2-[2-(4-Ethyl-phenyl)-1,1-dimethyl-ethylamino]-1-hydroxy-ethyl} -6-hydroxy-4H-benzo[1,4]oxazin-3-on
- 8-{2-[2-(4-Ethoxy-phenyl)-1,1-dimethyl-ethylamino]-1-hydroxy-ethyl}-6-hydroxy-4H-benzo[1,4]oxazin-3-on
- 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure
- 8-{2-[2-(3,4-Difluor-phenyl)-1,1-dimethyl-ethylamino]-1-hydroxy-ethyl}-6-hydroxy-4H-benzo[1,4]oxazin-3-on
- 1-(4-Ethoxy-carbonylamino-3-cyano-5-fluorophenyl)-2-(tert.-butylamino)ethanol
- 2-Hydroxy-5-(1-hydroxy-2-{2-[4-(2-hydroxy-2-phenyl-ethylamino)-phenyl]-ethylamino}-ethyl)-benzaldehyd
- N-[2-Hydroxy-5-(1-hydroxy-2-{2-[4-(2-hydroxy-2-phenyl-ethylamino)-phenyl]-ethylamino}-ethyl)-phenyl]-formamid
- 8-Hydroxy-5-(1-hydroxy-2-{2-[4-(6-methoxy-biphenyl-3-ylamino)-phenyl]-ethylamino}-ethyl)-1H-quinolin-2-on
- 8-Hydroxy-5-[1-hydroxy-2-(6-phenethylamino-hexylamino)-ethyl]-1H-quinolin-2-on
- 5-[2-(2-{4-[4-(2-Amino-2-methyl-propoxy)-phenylamino]-phenyl}-ethylamino)-1-hydroxy-ethyl]-8-hydroxy-1H-quinolin-2-on
- [3-(4-{6-[2-Hydroxy-2-(4-hydroxy-3-hydroxymethyl-phenyl)-ethylamino]-hexyloxy}-butyl)-5-methyl-phenyl]-harnstoff
- 4-(2-{6-[2-(2,6-Dichloro-benzyloxy)-ethoxy]-hexylamino}-1-hydroxy-ethyl)-2-hydroxymethyl-phenol
- 3-(4-{6-[2-Hydroxy-2-(4-hydroxy-3-hydroxymethyl-phenyl)-ethylamino]-hexyloxy} -butyl)-benzylsulfonamid
- 3-(3-{7-[2-Hydroxy-2-(4-hydroxy-3-hydroxymethyl-phenyl)-ethylamino]-heptyloxy}-propyl)-benzylsulfonamid
- 4-(2-{6-[4-(3-Cyclopentanesulfonyl-phenyl)-butoxy]-hexylamino}-1-hydroxy-ethyl)-2-hydroxymethyl-phenol
- N-Adamantan-2-yl-2-(3-{2-[2-hydroxy-2-(4-hydroxy-3-hydroxymethyl-phenyl)-ethylamino]-propyl}-phenyl)-acetamid
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Anticholinergika gelangen hierbei vorzugsweise Verbindungen zur Anwendung, die ausgewählt sind aus der Gruppe bestehend aus Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Glycopyrroniumsalzen, bevorzugt das Bromidsalz, Trospiumsalzen, bevorzugt das Chloridslz, Tolterodin. In den vorstehend genannten Salzen stellen die Kationen die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt.

Ebenfalls bevorzugte Anticholinergika sind ausgewählt aus den Salzen der Formel AC-1 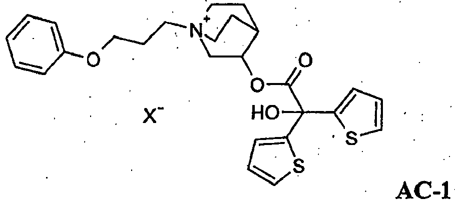 worin X⁻ ein einfach negativ geladenes Anion, bevorzugt ein Anion ausgewählt aus der Gruppe bestehend aus Fluorid, Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, bevorzugt ein einfach negativ geladenes Anion, besonders bevorzugt ein Anion ausgewählt aus der Gruppe bestehend aus Fluorid, Chlorid, Bromid, Methansulfonat und p-Toluolsulfonat, insbesondere bevorzugt Bromid, bedeutet gegebenenfalls in Form ihrer Racemate, Enantiomere oder Hydrate. Von besonderer Bedeutung sind solche Arzneimittelkombinationen, die die Enantiomere der Formel **AC-1-en** 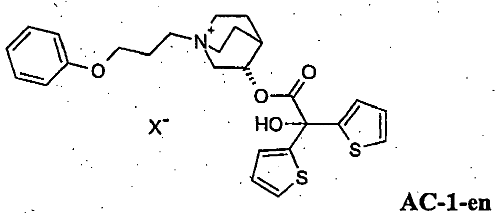 enthalten, worin X⁻ die vorstehend genannten Bedeutungen aufweisen kann. Weiterhin bevorzugte Anticholinergika sind ausgewählt aus den Salzen der Formel **AC-2** 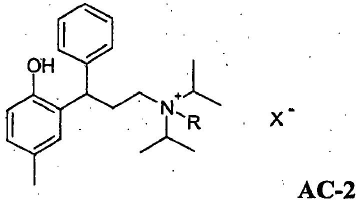 worin R entweder Methyl oder Ethyl bedeuten und worin X⁻ die vorstehend genannte Bedeutungen aufweisen kann. In einer alternativen Ausführungsform kann die Verbindung der Formel AC-2 auch in Form der freien Base **AC-2-base** vorliegen.

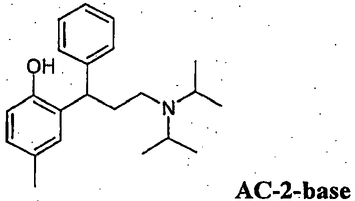

Weiterhin genannte Verbindungen sind:
- 2,2-Diphenylpropionsäuretropenolester-Methobromid
- 2,2-Diphenylpropionsäurescopinester-Methobromid
- 2-Fluor-2,2-Diphenylessigsäurescopinester-Methobromid
- 2-Fluor-2,2-Diphenylessigsäuretropenolester-Methobromid
- 3,3',4,4'-Tetrafluorbenzilsäuretropenolester-Methobromid
- 3,3',4,4'-Tetrafluorbenzilsäurescopinester-Methobromid
- 4,4'-Difluorbenzilsäuretropenolester-Methobromid
- 4,4'-Difluorbenzilsäurescopinester-Methobromid
- 3,3'-Difluorbenzilsäuretropenolester-Methobromid
- 3,3'-Difluorbenzilsäurescopinester-Methobromid
- 9-Hydroxy-fluoren-9-carbonsäuretropenolester-Methobromid
- 9-Fluor-fluoren-9-carbonsäuretropenolester-Methobromid
- 9-Hydroxy-fluoren-9-carbonsäurescopinester-Methobromid
- 9-Fluor-fluoren-9-carbonsäurescopinester-Methobromid
- 9-Methyl-fluoren-9-carbonsäuretropenolester-Methobromid
- 9-Methyl-fluoren-9-carbonsäurescopinester-Methobromid
- Benzilsäurecyclopropyltropinester-Methobromid
- 2,2-Diphenylpropionsäurecyclopropyltropinester-Methobromid
- 9-Hydroxy-xanthen-9-carbonsäurecyclopropyltropinester-Methobromid
- 9-Methyl-fluoren-9-carbonsäurecyclopropyltropinester-Methobromid
- 9-Methyl-xanthen-9-carbonsäurecyclopropyltropinester-Methobromid
- 9-Hydroxy-fluoren-9-carbonsäurecyclopropyltropinester-Methobromid
- 4,4'-Difluorbenzilsäuremethylestercyclopropyltropinester-Methobromid
- 9-Hydroxy-xanthen-9-carbonsäuretropenolester-Methobromid
- 9-Hydroxy-xanthen-9-carbonsäurescopinester-Methobromid
- 9-Methyl-xanthen-9-carbonsäuretropenolester-Methobromid
- 9-Methyl-xanthen-9-carbonsäurescopinester-Methobromid
- 9-Ethyl-xanthen-9-carbonsäuretropenolester-Methobromid
- 9-Difluormethyl-xanthen-9-carbonsäuretropenolester-Methobromid
- 9-Hydroxymethyl-xanthen-9-carbonsäurescopinester-Methobromid

Die vorstehend genannten Verbindungen sind im Rahmen der vorliegenden Erfindung auch als Salze einsetzbar, in denen statt des Methobromids, die Salze Metho-X zur Anwendung gelangen, wobei X die vorstehend für X- genannten Bedeutungen haben kann.

Als Corticosteroide gelangen hierbei vorzugsweise Verbindungen zur Anwendung, die ausgewählt sind aus der Gruppe bestehend aus Beclomethason, Betamethason, Budesonid, Butixocort, Ciclesonid, Deflazacort, Dexamethason, Etiprednol, Flunisolid, Fluticason, Loteprednol, Mometason, Prednisolon, Prednison, Rofleponid, Triamcinolon, RPR-106541, NS-126, ST-26 und
- 6,9-Difluor-17-[(2-furanylcarbonyl)oxy]-11-hydroxy-16-methyl-3-oxo-androsta-1,4-dien-17-carbothionsäure (S)-fluoromethylester
- 6,9-Difluor-11-hydroxy-16-methyl-3-oxo-17-propionyloxy-androsta-1,4-dien-17-carbothionsäure (S)-(2-oxo-tetrahydro-furan-3S-yl)ester,
- 6α,9α-difluoro-11β-hydroxy-16α-methyl-3-oxo-17α-(2,2,3,3-tertamethylcyclopropylcarbonyl)oxy-androsta-1,4-diene-17β-carbonsäure cyanomethyl ester
gegebenenfalls in Form ihrer Racemate, Enantiomere oder Diastereomere und gegebenenfalls in Form ihrer Salze und Derivate, ihrer Solvate und/oder Hydrate.

Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Dichloroacetate, Propionate, Dihydrogenphosphate, Palmitäte, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), Tofimilast, Pumafentrin, Lirimilast, Arofyllin, Atizoram, D-4418, Bay-198004, BY343, CP-325,366, D-4396 (Sch-351591), AWD-12-281 (GW-842470), NCS-613, CDP-840, D-4418, PD-168787, T-440, T-2585, V-11294A, C1-1018, CDC-801, CDC-3052, D-22888, YM-58997, Z-15370 und
- N-(3,5-Dichloro-1-oxo-pyridin-4-yl)-4-difluormethoxy-3-cyclopropylmethoxybenzamid
- (-)p-[(4*a*R*,10*b*S*)-9-Ethoxy-1,2,3,4,4a,10b-hexahydro-8-methoxy-2-methylbenzo[s][1,6]naphthyridin-6-yl]-N,N-diisopropylbenzamid
- (R)-(+)-1-(4-Brombenzyl)-4-[(3-cyclopentyloxy)-4-methoxyphenyl]-2-pyrrolidon
- 3-(Cyclopentyloxy-4-methoxyphenyl)-1-(4-N'-[N-2-cyano-S-methyl-isothioureido]benzyl)-2-pyrrolidon
- cis[4-Cyano-4-(3-cyclopentyloxy-4-methoxyphenyl)cyclohexan-1-carbonsäure]
- 2-carbomethoxy-4-cyano-4-(3-cyclopropylmethoxy-4-difluoromethoxy-phenyl)cyclohexan-1-on
- cis[4-Cyano-4-(3-cyclopropylmethoxy-4-difluormethoxyphenyl)cyclohexan-1-ol]
- (R)-(+)-Ethyl[4-(3-cyclopentyloxy-4-methoxyphenyl)pyrrolidin-2-yliden]acetat
- (S)-(-)-Ethyl[4-(3-cyclopentyloxy-4-methoxyphenyl)pyrrolidin-2-yliden]acetat
- 9-Cyclopentyl-5,6-dihydro-7-ethyl-3-(2-thienyl)-9*H*-pyrazolo[3,4-c]-1,2,4-triazolo[4,3-a]pyridin
- 9-Cyclopentyl-5,6-dihydro-7-ethyl-3-(*tert*--butyl)-9*H*-pyrazolo[3,4-c]-1,2,4-triazolo[4,3-a]pyridin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der PDE4-Inhibitoren ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321 und
- 1-(((R)-(3-(2-(6,7-Difluor-2-quinolinyl)ethenyl)phenyl)-3-(2-(2- hydroxy-2-propyl)phenyl)thio)methylcyclopropan-essigsäure,
- 1-(((1(R)-3(3-(2-(2,3-Dichlorthieno[3,2-b]pyridin-5-yI)-(E)-ethenyl)phenyl)-3-(2-(1-hydroxy-1-methylethyl)phenyl)-propyl)thio)methyl)cyclopropanessigsäure
- [2-[[2-(4-tert-Butyl-2-thiazolyl)-5-benzofuranyl]oxymethyl]phenyl]-essigsäure
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als EGFR-Hemmer gelangen hierbei vorzugsweise Verbindungen zur Anwendung, die ausgewählt sind aus der Gruppe bestehend aus Cetuximab, Trastuzumab, ABX-EGF, Mab ICR-62 und
- 4-[(3-Chlor-4-fluorphenyl)amino]-6-{[4-(morpholin-4-yl)-1-oxo-2-buten-1-yl]amino} -7-cyclopropylmethoxy-chinazolin
- 4-[(3-Chlor-4-fluorphenyl)amino]-6-{[4-(N,N-diethylamino)-1-oxo-2-buten-1-yl]amino}-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopropylmethoxy-chinazolin
- 4-[(R)-(1-Phenyl-ethyl)amino]-6-{[4-(morpholin-4-yl)-1-oxo-2-buten-1-yl]-amino}-7-cyclopentyloxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin
- 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin
- 4-[(3-Chlor-4-fluor-phenyl)amino]-6-[2-((S)-6-methyl-2-oxo-morpholin-4-yl)-ethoxy]-7-methoxy-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopentyloxy-chinazolin
- 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin
- 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin
- 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin
- 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin
- 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin
- 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin
- 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin
- 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin
- 4-[(3-Ethinyl-phenyl)amino]-6,7-bis-(2-methoxy-ethoxy)-chinazolin
- 4-[(3-Chlor-4-fluorphenyl)amino]-7-[3-(morpholin-4-yl)-propyloxy]-6-[(vinylcarbonyl)amino]-chinazolin
- 4-[(R)-(1-Phenyl-ethyl)amino]-6-(4-hydroxy-phenyl)-7H-pyrrolo[2,3-d]pyrimidin
- 3-Cyano-4-[(3-chlor-4-fluorphenyl)amino]-6-{[4-(N,N-dimethylamino)-1-oxo-2-buten-1-yl]amino}-7-ethoxy-chinolin
- 4-{[3-Chlor-4-(3-fluor-benzyloxy)-phenyl]amino}-6-(5-{[(2-methansulfonyl-ethyl)amino]methyl}-furan-2-yl)chinazolin
- 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin
- 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin
- 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin
- 4-[(3-Ethinyl-phenyl)amino]-6-{[4-(5,5-dimethyl-2-oxo-morpholin-4-yl)-1-oxo-2-buten-1-yl]amino}-chinazolin
- 4-[(3-Chlor-4-fluor-phenyl)amino]-6-[2-(2,2-dimethyl-6-oxo-morpholin-4-yl)-ethoxy]-7-methoxy-chinazolin
- 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin
- 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin
- 4-[(3-Chlor-4-fluor-phenyl)amino]-6-{2-[4-(2-oxo-morpholin-4-yl)-piperidin-1-yl]-ethoxy}-7-methoxy-chinazolin
- 4-[(3-Chlor-4-fluor-phenyl)amino]-6-[1-(tert.-butyloxycarbonyl)-piperidin-4-yloxy]-7-methoxy-chinazolin
- 4-[(3-Chlor-4-fluor-phenyl)amino]-6-(trans-4-amino-cyclohexan-1-yloxy)-7-methoxy-chinazolin
- 4-[(3-Chlor-4-fluor-phenyl)amino]-6-(trans-4-methansulfonylamino-cyclohexan-1-yloxy)-7-methoxy-chinazolin
- 4-[(3-Chlor-4-fluor-phenyl)amino]-6-(tetrahydropyran-3-yloxy)-7-methoxy-chinazolin
- 4-[(3-Chlor-4-fluor-phenyl)amino]-6-(1-methyl-piperidin-4-yloxy)-7-methoxy-chinazolin
- 4-[(3-Chlor-4-fluor-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(methoxymethyl)carbonyl]-piperidin-4-yloxy}-7-methoxy-chinazolin
- 4-[(3-Chlor-4-fluor-phenyl)amino]-6-(piperidin-3-yloxy)-7-methoxy-chinazolin
- 4-[(3-Chlor-4-fluor-phenyl)amino]-6-[1-(2-acetylamino-ethyl)-piperidin-4-yloxy]-7-methoxy-chinazolin
- 4-[(3-Chlor-4-fluor-phenyl)amino]-6-(tetrahydropyran-4-yloxy)-7-ethoxy-chinazolin
- 4-[(3-Chlor-4-fluor-phenyl)amino]-6-((S)-tetrahydrofuran-3-yloxy)-7-hydroxy-chinazolin
- 4-[(3-Chlor-4-fluor-phenyl)amino]-6-(tetrahydropyran-4-yloxy)-7-(2-methoxy-ethoxy)-chinazolin
- 4-[(3-Chlor-4-fluor-phenyl)amino]-6-{trans-4-[(dimethylamino)sulfonylamino]-cyclohexan-1-yloxy}-7-methoxy-chinazolin
- 4-[(3-Chlor-4-fluor-phenyl)amino]-6-{trans-4-[(morpholin-4-yl)carbonylamino]-cyclohexan-1-yloxy}-7-methoxy-chinazolin
- 4-[(3-Chlor-4-fluor-phenyl)amino]-6-{trans-4-[(morpholin-4-yl)sulfonylamino]-cyclohexan-1-yloxy}-7-methoxy-chinazolin
- 4-[(3-Chlor-4-fluor-phenyl)amino]-6-(tetrahydropyran-4-yloxy)-7-(2-acetylamino-ethoxy)-chinazolin
- 4[(3-Chlor-4-fluor-phenyl)amino]-6-(tetrahydropyran-4-yloxy)-7-(2-methansulfonylamino-ethoxy)-chinazolin
- 4-[(3-Chlor-4-fluor-phenyl)amino]-6-{1-[(piperidin-1-yl)carbonyl]-piperidin-4-yloxy}-7-methoxy-chinazolin
- 4-[(3-Chlor-4-fluor-phenyl)amino]-6-(1-aminocarbonylmethyl-piperidin-4-yloxy)-7-methoxy-chinazolin
- 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)sulfonyl]-N-methyl-amino}-cyclohexan-1-yloxy)-7-methoxy- chihazolin
- 4-[(3-Chlor-4-fluor-phenyl)amino]-6-(trans-4-ethansulfonylamino-cyclohexan-1-yloxy)-7-methoxy-chinazolin
- 4-[(3-Chlor-4-fluor-phenyl)amino]-6-(1-methansulfonyl-piperidin-4-yloxy)-7-ethoxy-chinazolin
- 4-[(3-Chlor-4-fluor-phenyl)amino]-6-(1-methansulfonyl-piperidin-4-yloxy)-7-(2-methoxy-ethoxy)-chinazolin
- 4-[(3-Chlor-4-fluor-phenyl)amino]-6-[1-(2-methoxy-acetyl)-piperidin-4-yloxy]-7-(2-methoxy-ethoxy)-chinazolin
- 4-[(3-Chlor-4-fluor-phenyl)amino]-6-(cis-4-acetylamino-cyclohexan-1-yloxy)-7-methoxy-chinazolin
- 4-[(3-Ethinyl-phenyl)amino]-6-[1-(tert.-butyloxycarbonyl)-piperidin-4-yloxy]-7-methoxy-chinazolin
- 4-[(3-Ethinyl-phenyl)amino]-6-(tetrahydropyran-4-yloxy]-7-methoxy-chinazolin
- 4-[(3-Chlor-4-fluor-phenyl)amino]-6-(cis-4-{N-[(piperidin-1-yl)carbonyl]-N-methyl-amino} -cyclohexan-1-yloxy)-7-methoxy-chinazolin
- 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin
- 4-[(3-Chlor-4-fluor-phenyl)amino]-6-{cis-4-[(morpholin-4-yl)carbonylamino]-cyclohexan-1-yloxy}-7-methoxy-chinazolin
- 4-[(3-Chlor-4-fluor-phenyl)amino]-6-{1-[2-(2-oxopyrrolidin-1-yl)ethyl]-piperidin-4-yloxy}-7-methoxy-chinazolin
- 4-[(3-Chlor-4-fluor-phenyl)amino]-6-{1-[(morpholin-4-yl)carbonyl]-piperidin-4-yloxy}-7-(2-methoxy-ethoxy)-chinazolin
- 4-[(3-Ethinyl-phenyl)amino]-6-(1-acetyl-piperidin-4-yloxy)-7-methoxy-chinazolin
- 4-[(3-Ethinyl-phenyl)amino]-6-(1-methyl-piperidin-4-yloxy)-7-methoxy-chinazolin
- 4-[(3-Ethinyl-phenyl)amino]-6-(1-methansulfonyl-piperidin-4-yloxy)-7-methoxy-chinazolin
- 4-[(3-Chlor-4-fluor-phenyl)amino]-6-(1-methyl-piperidin-4-yloxy)-7(2-methoxy-ethoxy)-chinazolin
- 4-[(3-Chlor-4-fluor-phenyl)amino]-6-(1-isopropyloxycarbonyl-piperidin-4-yloxy)-7-methoxy-chinazolin
- 4-[(3-Chlor-4-fluor-phenyl)amino]-6-(cis-4-methylamino-cyclohexan-1-yloxy)-7-methoxy-chinazolin
- 4-[(3-Chlor-4-fluor-phenyl)amino]-6-{cis-4-[N-(2-methoxy-acetyl)-N-methyl-amino]-cyclohexan-1-yloxy}-7-methoxy-chinazolin
- 4-[(3-Ethinyl-phenyl)amino]-6-(piperidin-4-yloxy)-7-methoxy-chinazolin
- 4-[(3-Ethinyl-phenyl)amino]-6-[1-(2-methoxy-acetyl)-piperidin-4-yloxy]-7-methoxy-chinazolin
- 4-[(3-Ethinyl-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(cis-2,6-dimethyl-morpholin-4-yl)carbonyl]-piperidin-4-yloxy}-7-methoxy-chinazolin
- 4-[(3-Chlor-4-fluor-phenyl)amino]-6-{1-[(2-methyl-morpholin-4-yl)carbonyl]-piperidin-4-yloxy}-7-methoxy-chinazolin
- 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin
- 4-[(3-Chlor-4-fluor-phenyl)amino]-6-{1-[(N-methyl-N-2-methoxyethyl-amino)carbonyl]-piperidin-4-yloxy}-7-methoxy-chinazolin
- 4-[(3-Chlor-4-fluor-phenyl)amino]-6-(1-ethyl-piperidin-4-yloxy)-7-methoxy-chinazolin
- 4-[(3-Chlor-4-fluor-phenyl)amino]-6-{1-[(2-methoxyethyl)carbonyl]-piperidin-4-yloxy}-7-methoxy-chinazolin
- 4-[(3-Chlor-4-fluor-phenyl)amino]-6-{1-[(3-methoxypropyl-amino)-carbonyl]-piperidin-4-yloxy}-7-methoxy-chinazolin
- 4-[(3-Chlor-4-fluor-phenyl)amino]-6-[cis-4-(N-methansulfonyl-N-methyl-amino)-cyclohexan-1-yloxy]-7-methoxy-chinazolin
- 4-[(3-Chlor-4-fluor-phenyl)amino]-6-[cis-4-(N-acetyl-N-methyl-amino)-cyclohexan-1-yloxy]-7-methoxy-chinazolin
- 4-[(3-Chlor-4-fluor-phenyl)amino]-6-(trans-4-methylamino-cyclohexan-1-yloxy)-7-methoxy-chinazolin
- 4-[(3-Chlor-4-fluor-phenyl)amino]-6-[trans-4-(N-methansulfonyl-N-methyl-amino)-cyclohexan-1-yloxy]-7-methoxy-chinazolin
- 4-[(3-Chlor-4-fluor-phenyl)amino]-6-(trans-4-dimethylamino-cyclohexan-1-yloxy)-7-methoxy-chinazolin
- 4-[(3-Chlor-4-fluor-phenyl)amino]-6-(trans-4- {N-[(morpholin-4-yl)carbonyl]-N-methyl-amino}-cyclohexan-1-yloxy)-7-methoxy-chinazolin
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin
- 4-[(3-Chlor-4-fluor-phenyl)amino]-6-(1-methansulfonyl-piperidin-4-yloxy)-7-methoxy-chinazolin
- 4-[(3-Chlor-4-fluor-phenyl)amino]-6-(1-cyano-piperidin-4-yloxy)-7-methoxy-chinazolin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus-Hydrocholrid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Dopamin-Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Außerdem können inhalierbare Makromoleküle verwendet werden, wie in EP 1 003 478 offenbart.

Weiterhin kann die Verbindung aus der Gruppe der Derivate von Mutterkornalkaloiden, der Triptane, der CGRP-Hemmern, der Phosphodiesterase-V-Hemmer stammen, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Als Derivate der Mutterkornalkaloide: Dihydroergotamin, Ergotamin.

Für die Inhalation kommen Arzneimittel, Arzneimittelformulierungen und - mischungen mit den o.g. Wirkstoffen in Betracht, sowie deren Salze, Ester sowie die Kombination dieser Wirkstoffe, Salze und Ester.

Im Folgenden ist die Erfindung anhand von vereinfachten, schematisch dargestellten Beispielen gezeigt. Dabei zeigt
Fig. 1 ein Medikamentenmagazin mit zwei Gruppen von Medikamentenkammern
Fig. 2 eine Medikamenten-Ausgabevorrichtung
Fig. 3 eine weitere Medikamenten-Ausgabevorrichtung

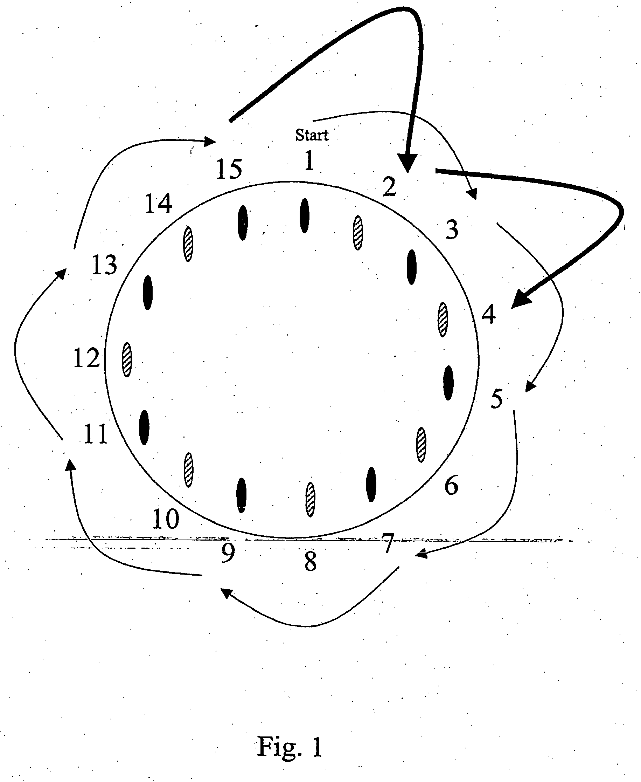

In **Figur 1** ist das Prinzip der erfindungsgemässen Medikamenten-Ausgabevorrichtung anhand von kreisförmig, äquidistant angeordneten Medikamentenkammern gezeigt. Die insgesamt 15 Medikamentenkammern sind in zwei Gruppen unterteilt. Die erste Gruppe von Medikamentenkammern sind in der Figur als gefüllte Ovale, die zweite Gruppe als gestrichelte Ovale dargestellt. Nachdem bei eine Medikamentenkammer Nr.1 gestartet wird und diese verbraucht ist, wird jeweils eine übernächste Kammer, Nr. 3, dann Nr.5 etc. angefahren, solange, bis sämtliche Kammern der Gruppe 1 abgefahren, d.h.. verbraucht sind. Die Position der letzten Kammer der Gruppe 1, hier Nr. 15, liegt benachbart zur Kammer Nr. 1, um eine halbe Vorschubdistanz entfernt. Mit derselben Vorschubdistanz wie bisher, z.B. durch eine Vorwärtsbewegung eines Mundsstücks oder eine entsprechende Bewegung des Magazin bzw. Gehäuses, wird die erste Kammer der zweiten Gruppe, Nr. 2, angefahren. Nun werden sämtliche Kammern der Gruppe 2 nacheinander angefahren, wobei jeweils die bereits verbrauchten Kammern der Gruppe 1 übersprungen werden.

Eine Medikamentenanzeige, welche anzeigt, die wievielte Kammer gerade benutzt wird, bzw. wieviele Kammern noch im Magazin verbleiben, ist vorzugsweise in der Reihenfolge numeriert, wie die Kammern angefahren werden.

In diesem Beispiels wird jeweils eine Medikamentenkammer übersprungen. Nach einer knappen Umdrehung, welche einer ganzen Umdrehung minus einer halben Vorschubdistanz entspricht, wird von einer ersten Gruppe von Medikamentenkammern in eine zweite Gruppe übergegangen.

An diesem Beispiel ist klar ersichtlich, dass je nach Vorschubdistanz und Anordnungsdichte der Medikamentenmagazine auch ein Überspringen von zwei, drei oder mehreren Medikamentenkammern erforderlich oder vorteilhaft ist. Entsprechend sind die Kammern in drei, vier, oder mehrere Gruppen unterteilt, wobei die letzte Kammer der ersten Gruppe vorzugsweise jeweils um weniger als eine Vorschubdistanz von der ersten Kammer der ersten Gruppe beabstandet ist.

Dieses Beispiel zeigt zudem eine äquidistante Anordnung sämtlicher Medikamentenkammern (aber nicht der einzelnen Gruppen) und einen darauf abgestimmten ebenfalls äquidistanten Vorschub. Eine solche Ausführungsform ist aufgrund der einfachen Geometrie für ein Medikamentenmagazin und aufgrund der kontinuierlichen Bewegung und einer entsprechend einfachen Mechanik für den Vorschub vorteilhaft.

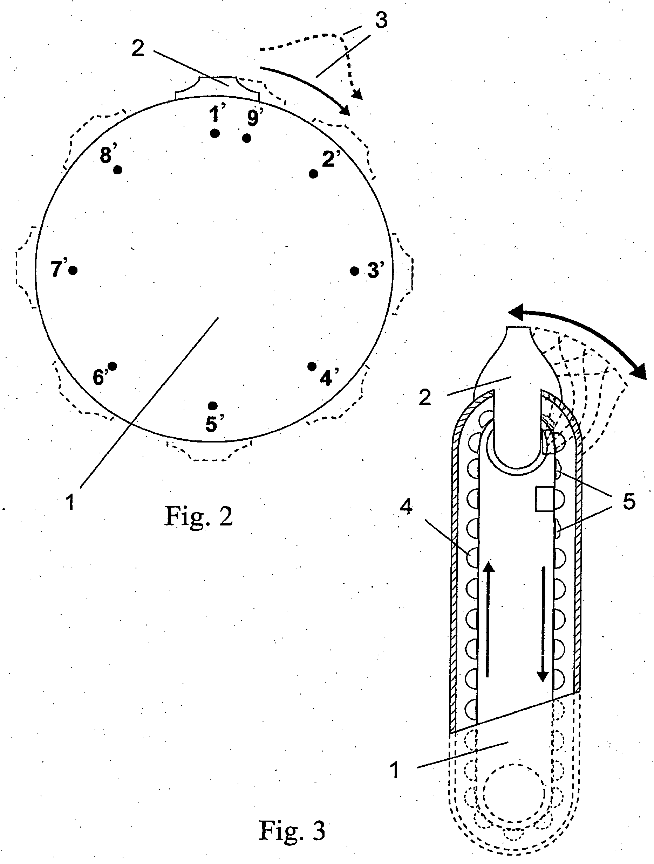

In **Figur 2****,** die eine von den Ansprüchen nicht umfasste Ausführungsform zeigt, ist ein rundes, scheibenförmiges Inhalationsgerät, beispielsweise ein Mehrdosispulverinhalator, mit einem an seinem Aussenumfang angebrachten Mundstück 2 gezeigt. Dabei beziffern die Zahlen 1' bis 9' die Medikamentenkammer- und Entnahmepositioncn in oder Reihenfolge, wie sie angefahren werden. Dazwischen liegende Kammerpositionen sind bis auf die Position Nr. 9' nicht eingezeichnet. In dieser Ausführungsform wird das Mundstück 2 jeweils um eine bestimmte Vorschubdistanz 3, hier mit Pfeilen eingezeichnet, mit der nächsten Medikamentenkammer in Übereinstimmung gebracht. Der dunklere, radial verlaufende Vorschub-pfeil entspricht im wesentlichen der Bewegungsrichtung des Mundstücks, während der gestrichelte, bogenförmig eingezeichnete Pfeil einer Vorschubdistanz eines Mundstück oder einer im Inneren befindlichen Mechanik entspricht, welche beispielsweise aufgrund eines Stechvorganges einen gewissen Hub vor der Positionierung vor einer neuen Medikamentenkammer aufweisen muss.

Zwischen der Position 8' und 9' liegt kein äaquidistanter Vorschub. Ein solcher Zusatzvorschub zwischen einer Gruppe und einer nächsten Gruppe kann mit einer entsprechenden Mechanik im Gehäuse 1 vorgenommen und gleichzeitig als Indikator verwendet werden. Ein solcher Indikator kann beispielsweise als zusätzlicher Hinweis zwischen einzelnen zu verabreichenden Indikationen, Dosierungen oder Medikamentenzusammensetzungen dienen, z. B 1. Woche/2. Woche, 1. Monat: niedrigere Dosierung/2. Monat höhere Dosierung oder 1.-10. Tag: erstes Medikament/11.-16.Tag: zweites Medikament.

Unterschiedliche Positionen des Mundstücks 2 in den Medikamentenkammerpositionen 2' bis 9' sind in der Figur gestrichelt gezeichnet.

In **Figur 3** ist eine Medikamenten-Ausgabevorrichtung mit einem raupenförmig eingebrachten endlos Blisterband 4 gezeigt. Über das Mundstück 2 wird das Blisterband weitertransportiert, wobei das Mundstück beweglich, aber im wesentlichen ortsfest am Gehäuse 1 angebracht ist. Auf der einen Seite des Mundstücks ist jeweils ein übernächster Blister als verbrauchte Medikamentenkammer 5 eingezeichnet. Das Medikamentenmagazin bewegt sich bezüglich des Mundstücks und Gehäuses, wobei verschiedene Mundstückpositionen gestrichelt eingezeichnet sind.

## Patentansprüche

1. Medikamenten-Ausgabevorrichtung zur Ausgabe von Medikamenten-Einzeldosen, mit einem Medikamentenmagazin und einer darin eingebrachten Vielzahl von Medikamentenkammern (5), wobei die Medikamentenkammern in der Form einer Endlosschlaufe im Medikamentenmagazin vorliegen, wobei die Medikamentenkammern äquidistant zueinander angeordnet sind und wobei das Medikamentenmagazin und ein Mundstück relativ zueinander verschiebbar angeordnet sind, derart dass die Medikamentenkammern und das Mundstück (2) miteinander in Übereinstimmung bringbar sind, **dadurch gekennzeichnet, dass** bei einem Vorschub des Mundstücks von einer Entnahmeposition zur nächsten Entnahmeposition das Mundstück über mehrere Medikamentenkammerpositionen hinweg bewegt wird und zwei so aufeinander folgende Medikamentenkammern in einer derartigen Distanz zueinander angeordnet sind, dass nach einem im wesentlichen vollständigen Umgang des Medikamentenmagazins, das Mundstück (2) in einer Entnahmeposition ist, welche einer Medikamentenkammerposition x + 1 oder x - 1 entspricht, bei einer anfänglichen Medikamentenkammerposition x.

2. Medikamenten-Ausgabevorrichtung nach Anspruch 1, wobei Mundstück (2) und Medikamentenmagazin nur in einer Richtung relativ zueinander verschiebbar angeordnet sind.

3. Medikamenten-Ausgabevorrichtung nach einem der Ansprüche 1 oder 2, wobei zwischen zwei aufeinanderfolgenden Entnahmepositionen, eine, zwei oder drei Medikamentenkammern (5) angeordnet sind.

4. Medikamenten-Ausgabevorrichtung nach einem der Ansprüche 1-3, wobei das Medikamentenmagazin in einem im wesentlichen scheibenförmigen Gehäuse untergebracht ist und das Mundstück (2) entlang einem Aussenumfang der Scheibe verschiebbar angeordnet ist.

5. Medikamenten-Ausgabevorrichtung nach einem der Ansprüche 1-4, wobei die Medikamentenkammern (5) ringförmig im Medikamentenmagazin angeordnet sind.

6. Medikamenten-Ausgabevorrichtung nach einem der Ansprüche 1-5, wobei die Medikamentenkammern (5) in einem endlos Blisterband (4) angeordnet sind.

7. Medikamenten-Ausgabevorrichtung nach einem der Ansprüche 1-6, mit einer Anzeige zum Anzeigen in welcher Medikamentenkammerposition und/oder Entnahmeposition sich das Mundstück (2) befindet.

8. Medikamenten-Ausgabevorrichtung nach einem der vorhergehenden Ansprüche, wobei bei einem Betätigen des Mundstücks (2), ein Medikamentenmagazin bezüglich eines Gehäuses (1) nicht verschoben wird.

9. Medikamenten-Ausgabevorrichtung nach einem der vorhergehenden Ansprüche, wobei die Vorrichtung ein Mehrdosispulverinhalator ist.

10. Medikamenten-Ausgabevorrichtung nach einem der vorhergehenden Ansprüche, wobei die Vielzahl der Medikamentenkammern (5) maximal 30 oder 60 beträgt.

11. Medikamenten-Ausgabevorrichtung nach einem der vorhergehenden Ansprüche zur Applikation eines Arzneimittels, das einen Wirkstoff oder eine Kombination der Wirkstoffe enthält ausgewählt aus der Gruppe Betamimetika, Anticholinergika, Steroide, Antiallergika, Derivate von Mutterkornalkaloiden, Triptane, CGRP-Antagonisten, der Phosphodiesterase -V-Inhibitoren, Phosphodiesterase-IV-Inhibitoren, LTD4-Antagonisten, EGFR-Kinase-Hemmer

## Claims

1. Medicine dispensing device for the dispensing of single doses of medicine, with a medicine magazine and a multitude of medicine chambers (5) arranged therein, wherein the medicine chambers are arranged in the form of a continuous loop within the medicine magazine, wherein the medicine chambers are equidistant to one another, and wherein the medicine magazine and a mouthpiece are arranged to be movable relative to each other so that each medicine chambers can be aligned to coincide with the mouthpiece (2), **characterized in that,** when the mouthpiece is forwarded from one removal position to a next removal position, the mouthpiece is moved across several medicine chamber positions and any two such consecutive medicine chambers are arranged at such a distance from one another that, after an essentially complete rotation of the medicine magazine, the mouthpiece (2), having started in a medicine chamber position x, is in a removal position corresponding with a medicine chamber position x + 1 or x - 1.

2. Medicine dispensing device according to claim 1, wherein the mouthpiece (2) and the medicine magazine can only be moved in one direction relative to each other.

3. Medicine dispensing device according to one of the claims 1 or 2, wherein one, two or three medicine chambers (5) are situated between two consecutive removal positions.

4. Medicine dispensing device according to any one of the claims 1 to 3, wherein the medicine magazine is accommodated in an essentially disc-shaped casing and the mouthpiece (2) is arranged to be movable along an outer circumference of the disc.

5. Medicine dispensing device according to any one of the claims 1 to 4, wherein the medicine chambers (5) are arranged along a ring in the medicine magazine.

6. Medicine dispensing device according to any one of the claims 1 to 5, wherein the medicine chambers (5) are arranged in an endless blister band (4).

7. Medicine dispensing device according to any one of the claims 1 to 6, with an indicator to indicate in which medicine chamber position and/or removal position the mouthpiece (2) is situated.

8. Medicine dispensing device according to any one of the aforementioned claims, wherein a medicine magazine is not shifted relative to the casing (1) when the mouthpiece (2) is operated.

9. Medicine dispensing device according to any one of the aforementioned claims, wherein the device is a multiple-dose powder inhalator.

10. Medicine dispensing device according to any one of the aforementioned claims, wherein the multitude of medicine chambers (5) does not exceed 30 or 60.

11. Medicine dispensing device according to any one of the aforementioned claims, for application of a drug that contains an active substance or a combination of active substances selected from the group of betamimetics, anticholinergics, steroids, antiallergens, derivatives of ergotalkaloids, triptane, CGRP-antagonists, phosphodiesterase-V-inhibitors, phosphodiesterase-IV-inhibitors, LTD4-antagonists, EGFR-kinase-inhibitors.

## Revendications

1. Distributeur de médicament servant à distribuer des doses unitaires de médicament, présentant un magasin à médicament dans lequel sont disposées plusieurs chambres (5) à médicament,
les chambres à médicament présentant la forme d'une boucle sans fin dans le magasin à médicament,
les chambres à médicament étant disposées à même distance les unes des autres,
le magasin à médicament et une pièce d'embouchure pouvant déplaçer l'un par rapport à l'autre de telle sorte que les chambres à médicament et la pièce d'embouchure (2) puissent être amenées à se correspondre mutuellement,
**caractérisé en ce que**
lorsque la pièce d'embouchure est avancée d'une position de prélèvement à une position de prélèvement suivante, la pièce d'embouchure est déplacée sur plusieurs positions de chambres à médicament et deux chambres à médicament successives sont disposées à une distance mutuelle telle qu'après avoir traversé essentiellement tout le magasin à médicament, la pièce d'embouchure (2) se trouve dans une position de prélèvement qui correspond à une position x + 1 ou x - 1 par rapport à une position initiale x de chambres à médicament.

2. Distributeur de médicament selon la revendication 1, dans lequel la pièce d'embouchure (2) et le magasin à médicament sont disposés de manière à ne pouvoir se déplacer mutuellement que en une direction.

3. Distributeur de médicament selon l'une des revendications 1 ou 2, dans lequel une, deux ou trois chambres (5) à médicament sont disposées entre deux positions de prélèvement successives.

4. Distributeur de médicament selon l'une des revendications 1 à 3, dans lequel le magasin à médicament est placé dans un boîtier essentiellement en forme de disque, la pièce d'embouchure (2) étant disposée de manière déplaçable le long de la périphérie extérieure du disque.

5. Distributeur de médicament selon l'une des revendications 1 à 4, dans lequel les chambres (5) à médicament sont disposées de façon d'un anneau dans le magasin à médicament.

6. Distributeur de médicament selon l'une des revendications 1 à 5, dans lequel les chambres (5) à médicament sont disposées dans une bande de blister (4) sans fin.

7. Distributeur de médicament selon l'une des revendications 1 à 6, présentant un affichage qui affiche la position de chambres à médicament et/ou la position de prélèvement dans laquelle se trouve la pièce d'embouchure (2).

8. Distributeur de médicament selon l'une des revendications précédentes, dans lequel un magasin à médicament n'est pas déplacé par rapport au boîtier (1) lorsque la pièce d'embouchure (2) est actionnée.

9. Distributeur de médicament selon l'une des revendications précédentes, dans lequel le distributeur est un inhalateur de poudre multidoses.

10. Distributeur de médicament selon l'une des revendications précédentes, dans lequel le nombre de chambres (5) à médicament est d'au plus 30 ou 60.

11. Distributeur de médicament selon l'une des revendications précédentes, pour l'application d'un médicament qui contient une substance active ou une combinaison de substances actives sélectionnées dans l'ensemble : des bêtamimétiques, des anticholinergiques, des stéroïdes, des antiallergiques, des dérivés des alcaloïdes de l'ergot, des triptanes, des antagonistes CGRP, des inhibiteurs de phosphodiestérase-V, des inhibiteurs de phosphodiestérase-IV, des antagonistes LTD4 et des inhibiteurs d'EGFR-kinase.
